# EUROPÄISCHE PATENTSCHRIFT

(11) **EP 1 374 837 B1**
(45) Veröffentlichungstag und Bekanntmachung des Hinweises auf die Patenterteilung: **01.03.2006**
(21) Anmeldenummer: 03011977.0
(22) Anmeldetag: 28.05.2003
(51) Int. Cl.: A61K 8/04, A61K 8/34, A61K 8/81, A61Q 5/06

(54) **Aerosolschaumprodukt zur Haarbehandlung**
Aerosol foam for treating the hair
Mousse aérosol pour traitement capillaire

(30) Priorität: 26.06.2002 DE 10228436
(43) Veröffentlichungstag der Anmeldung: 02.01.2004
(73) Patentinhaber: Wella Aktiengesellschaft, 64274 Darmstadt (DE)
(72) Erfinder: Birkel, Susanne, Dr., 64285 Darmstadt (DE); Wendel, Harald, 64372 Ober-Ramstadt (DE); Franzke, Michael, Dr., 64380 Rossdorf (DE); Henze, Hildegard, 64297 Darmstadt (DE); Hannich, Manuela, 63329 Egelsbach (DE)

(56) Entgegenhaltungen:
- WO-A-98/16653
- WO-A-99/25311
- DE-A- 3 217 059
- US-A- 4 240 450

## Beschreibung

Gegenstand der Erfindung ist ein Aerosolschaumprodukt zur Haarbehandlung umfassend eine Zusammensetzung mit einem Gehalt an an einem Dialkyldiallylammoniumchlorid/Acrylamid Copolymer und einem teilweise oder vollständig neutralisiertem Copolymer aus Alkylacrylat oder Alkylmethacrylat und Acrylsäure oder Methacrylsäure.

Schäume zur Haarbehandlung sind bekannt und bestehen in der Regel aus haarfestigenden oder haarpflegenden Substanzen, Schaumbildnern sowie einer geeigneten, in der Regel wasser-haltigen Lösungsmittelbasis. Die Zusammensetzungen werden mittels eines Treibgases oder mittels einer mechanisch betriebenen Pumpschaumvorrichtung unmittelbar vor der Anwendung verschäumt. An die Qualität von derartigen Schaumprodukten werden verschiedene Anforderungen gestellt, die grob in zwei Gruppen aufgeteilt werden können. Die eine Gruppe von Qualitätsanforderungen betrifft die Schaumqualität, d.h. die Beschaffenheit des Schaumes. Hierzu gehören etwa die Fein-oder Grobporigkeit, die Kompaktheit, die zeitliche Stabilität des Schaumes, die Einarbeitbarkeit und Verteilbarkeit auf dem Haar etc.. Die zweite Gruppe von Qualitätsanforderungen betrifft die Wirkungen, die der Schaum nach Einarbeitung in das Haar auf dem Haar hervorruft, d.h. die haarpflegenden Eigenschaften wie z.B. der Griff des Haares im feuchten und trockenen Zustand, die Kämmbarkeit, die Festigung, die Belastung des Haares, den Glanz des Haares etc..

Bei der Optimierung von Schaumprodukten besteht das Problem, dass zwar durch Zusatz spezieller Wirkstoffe eine Verbesserung einiger Qualitätsanforderungen, beispielsweise der Schaumqualität, erreicht werden kann, dies aber mit einer Verschlechterung anderer Qualitätsanforderungen, beispielsweise der haarpflegenden oder haarfestigenden Eigenschaften, erkauft wird. Häufig sind in Schaumprodukten kationische Polymere aufgrund der guten Substantivität zu menschlichem Haar enthalten.

Aus der DE 32 17 059 sind Aerosolschäume bekannt mit einem Gehalt an einer Kombination von bestimmten kationischen und anionischen Polymeren. Aus der WO 99/25311 sind Non-Aerosol Pumpschäume bekannt mit einem Gehalt an einer Kombination von bestimmten kationischen und anionischen Polymeren. Es bestand die Aufgabe, die Qualitätsanforderungen von Schaumprodukten zur Haarbehandlung hinsichtlich Schaumqualität und haarpflegenden bzw. haarfestigenden Eigenschaften weiter zu optimieren und zu verbessern.

Es wurde nun gefunden, dass die Aufgabe durch das nachfolgend beschriebene Schaumprodukt gelöst wird. Dabei wird unter einem Aerosolschaumprodukt ein Produkt verstanden, welches aus einer fluiden, verschäumbaren Zusammensetzung besteht, welche zusammen mit einem Treibgas in einer druckdichten Verpackung abgefüllt ist, die Verpackung mit einem Schaumkopf versehen ist und die Zusammensetzung unmittelbar vor der Anwendung in Form eines Schaumes ausgebracht werden kann. Gegenstand der Erfindung ist ein Aerosolschaumprodukt zur Haarbehandlung, umfassend eine Zusammensetzung mit einem Gehalt an
(A) mindestens einem Dialkyldiallylammoniumchlorid/Acrylamid Copolymer,
(B) mindestens einem Copolymer, gebildet aus einer ersten Monomerart, die ausgewählt ist aus Alkylacrylaten und Alkylmethacrylaten und mindestens einer zweiten Monomerart, die ausgewählt ist aus Acrylsäure und Methacrylsäure,
(C) mindestens einer Base zur teilweisen oder vollständigen Neutralisation des Copolymers (B) und
(D) einem geeigneten Lösungsmittelsystem,
   wobei die Zusammensetzung zusammen mit mindestens einem Treibmittel (E) in einer druckfesten Verpackung abgefüllt ist.

Das Copolymer (A) ist in der erfindungsgemäßen Zusammensetzung vorzugsweise in einer Menge von 0,01 bis 10, besonders bevorzugt von 0,1 bis 5 Gew.% enthalten. Das Copolymer (B) ist in der erfindungsgemäßen Zusammensetzung vorzugsweise in einer Menge von 0,1 bis 10, besonders bevorzugt von 0,5 bis 5 Gew.% enthalten. Das Gewichtsverhältnis des Copolmyers (B) zum Copolymer (A) liegt vorzugsweise im Bereich von größer 1:1 bis kleiner 10:1, besonders bevorzugt im Bereich von 2:1 bis 5:1. Das Verhältnis der Anzahl negativer Ladungen des Copolymers (B) zur Anzahl positiver Ladungen des Copolymer (A) liegt vorzugsweise im Bereich von 1:1 bis 10:1, vorzugsweise 3:1 bis 8:1.

In einer besonderen Ausführungsform hat der durch Verschäumen der erfindungsgemäßen Zusammensetzung gebildete Schaum fädenziehende Eigenschaften. Gegenstand der Erfindung ist daher auch ein Aerosolschaumprodukt zur Haarbehandlung, umfassend eine verschäumbare Zusammensetzung mit einem Gehalt an mindestens einem haarfestigenden Polymer, mindestens einem Treibmittel und einem geeigneten Lösungsmittelsystem, abgefüllt in einer druckfesten Verpackung, wobei der sich nach Abgabe aus der Verpackung sich bildende Schaum fädenziehende Eigenschaften aufweist. Fädenziehende Eigenschaften werden erkennbar, wenn etwas frisch hergestellter Schaum zwischen zwei Finger, z.B. Daumen und Zeigefinger, genommen wird und die Finger auseinander bewegt werden und sich spinnwebartige Fäden eines Durchmessers von etwa 2 mm, vorzugsweise kleiner 1 mm ausbilden. Fäden können auch erkennbar werden, wenn der Schaum auf eine Unterlage aufgebracht wird, die Schaumoberfläche mit einem Finger berührt wird und der Finger vom Schaum weggezogen wird. Die sehr guten Anwendungseigenschaften des Schaums nach Einarbeitung in das Haar können vermutlich u.a. auf die fädenziehenden Eigenschaften und die dadurch verbesserte Vernetzung der Haare zurückgeführt werden.

Das erfindungsgemäße Schaumprodukt zeichnet sich bei einer Anwendung auf menschlichen Haaren zur Frisurerstellung insbesondere durch eine gute Feuchtkämmbarkeit, gute Föneigenschaften, gute Formerstellung, gute Lockendefinition, gute Griffeigenschaften, insbesondere einen glatten Griff, und eine reduzierte Belastung aus. Gute Föneigenschaften bedeutet, dass, nachdem feuchtes Haar mit dem erfindungsgemäßen Produkt behandelt wurde, das Gleitvermögen der Haarbürste im Haar während des Fönens verbessert ist.

Aufgrund der guten Lockendefinition eignet sich die erfindungsgemäße Zusammensetzung besonders zur Behandlung von gelockten oder gewellten Haaren. Gegenstand der Erfindung ist daher auch die Verwendung einer Zusammensetzung mit einem Gehalt an (A) mindestens einem Dialkyldiallylammoniumchlorid/Acrylamid Copolymer, (B) mindestens einem Copolymer, gebildet aus einer ersten Monomerart, die ausgewählt ist aus Alkylacrylaten und Alkylmethacrylaten und mindestens einer zweiten Monomerart, die ausgewählt ist aus Acrylsäure und Methacrylsäure, (C) mindestens einer Base zur teilweisen oder vollständigen Neutralisation des Copolymers (B) und (D) einem geeigneten Lösungsmittelsystem zur Behandlung oder Herstellung gelockter oder gewellter Haare (Lockendefinition).

### Dialkyldiallylammoniumchlorid/Acrylamid Copolymer (A)

Die Alkylgruppen des Dialkyldiallylammoniumchlorid/Acrylamid Copolymers (A) weisen vorzugsweise 1 bis 4 C-Atome auf. Besonders bevorzugt sind Methylgruppen. Geeignete Dimethyldiallylammoniumchlorid/Acrylamid Copolymere haben die INCI-Bezeichnung Polyquaternium-7 und werden unter der Handelsbezeichnung Merquat® vertrieben. Bevorzugt sind Polymere mit einem Molekulargewicht im Bereich von 500.000 bis 20.000.000, insbesondere von 1.000.000 bis 10.000.000. Bevorzugte kationische Ladungsdichten der Polymere, ausgedrückt als Molekulargewicht pro kationische Ladung liegen im Bereich von 200 bis 250. Geeignet sind z.B. Merquat® 550 oder Merquat® 550 L.

### Alkyl(meth)acrylat/(Meth)acrylsäure Copolymer (B)

Das Copolymer (B) wird gebildet aus mindestens einer ersten Monomerart, die ausgewählt ist aus Alkylacrylaten und Alkylmethacrylaten und mindestens einer zweiten Monomerart, die ausgewählt ist aus Acrylsäure und Methacrylsäure. Die Alkylgruppen weisen vorzgusweise 1 bis 4 C-Atome aus, z.B. Methyl, Ethyl, Propyl, Isopropyl oder Butyl. Bevorzugte Copolymere sind Alkylacrylat/Methacrylsäure Copolymere. Geeignet ist z.B. Ethylacrylat/Methacrylsäure Copolymer (INCI-Bezeichnung Acrylates Copolymer) und wird unter der Handelsbezeichnung Luviflex® Soft vertrieben.

### Neutralisationsmittel (C)

Das Copolymer (B) liegt in vollständig oder partiell neutralisierter Form vor. Der Neutralisationsgrad liegt vorzugsweise bei mindestens 50%, besonders bevorzugt zwischen 70 und 100%, bezogen auf die Anzahl der freien Säuregruppen. Als Neutralisationsmittel können prinzipiell alle kosmetisch verträglichen anorganischen oder organischen Basen verwendet werden. Beispiele für Basen sind insbesondere Aminoalkanole wie z.B. Aminomethylpropanol (AMP), Triethanolamin oder Monoethanolamin, aber auch Ammoniak, NaOH, KOH u.a., wobei die Aminoalkanole, insbesondere AMP bevorzugt sind.

### Lösungsmittelsystem (D)

Die erfindungsgemäße Zusammensetzung wird in einem rein wässrigen oder in einem wässrig-alkoholischen Medium mit vorzugsweise mindestens 50 Gew.% Wasser und mindestens 5 Gew.% Alkohol konfektioniert. Das Lösungsmittelsystem ist vorzugsweise in einer Menge von 50 bis 98, besonders bevorzugt von 75 bis 95 Gew.% enthalten. Als Alkohole können insbesondere die für kosmetische Zwecke üblicherweise verwendeten niederen Alkohole mit 1 bis 4 C-Atomen, z.B. Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol.

### Treibmittel (E)

Das Treibmittel (E) ist in dem erfindungsgemäßen Aersolhaarschaum vorzugsweise in einer Menge von 1 bis 20, besonders bevorzugt von 2 bis 10 Gew.% enthalten. Als Treibmittel sind beispielsweise niedere Alkane, z.B. n-Butan, i-Butan, Propan, Butan oder deren Gemische sowie Dimethylether oder Fluorkohlenwasserstoffe wie 1,1-Difluorethan oder Tetrafluorethan sowie ferner bei den in Betracht kommenden Drücken gasförmig vorliegende Treibmittel, z.B. N₂, N₂O und CO₂ sowie Gemische der vorstehend genannten Treibmittel geeignet. Besonders bevorzugt sind C3- bis C4-Kohlenwasserstoffe.

### Tensid (F)

In einer bevorzugten Ausführungsform ist in dem erfindungsgemäßen Schaumprodukt zusätzlich mindestens ein Tensid (F) enthalten. Das Tensid (F) ist vorzugsweise in einer Menge von 0,01 bis 15, besonders bevorzugt von 0,05 bis 10 Gew.% enthalten. Geeignete Tenside haben emulgierende, lösungsvermittelnde, schaumbildende, schaumverstärkende oder haarpflegende Eigenschaften, sind vorzugsweise kationisch oder nichtionisch und haben einem HLB-Wert von maximal 20, vorzugsweise von 5 bis 18. In einer besonders bevorzugten Ausführungsform ist sowohl ein nichtionisches als auch ein kationisches Tensid enthalten.

Die nichtionischen Tenside können ausgewählt sein aus Fettsäureglyceridethoxylaten, Fettalkoholethoxylaten, Fettaminethoxylaten, Fettsäurealkanolamidethoxylaten, Fettsäureesterethoxylaten, ethoxylierten Fettsäurezuckerestern, ethoxylierten Sorbitanfettsäureestern, nicht-ethoxylierten Fettsäurezuckerestern und Alkylpolyglycosiden.
Bevorzugte nichtionische Tenside sind ethoxylierte Tenside, wobei die Anzahl der Ethylenoxid-Einheiten zwischen 1 bis 1000, bevorzugt zwischen 1 bis 300, besonders bevorzugt zwischen 1 und 15 liegt. Bevorzugt werden Fettsäureglyceridethoxylate, Fettalkoholethoxylate, Fettaminethoxylate, Fettsäurealkanolamidethoxylate und Fettsäureesterethoxylate mit jeweils 1 bis 50 Ethylenoxideinheiten. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet. Unter den Fettsäureesterethoxylaten sind vor allem Diglyceridethoxylate zu nennen, z.B. mit 25 Ethylenoxid-Einheiten ethoxyliertes Rizinusöl mit dem INCI-Namen PEG-25 Hydrogenated Castor Oil (Arlatone® G), mit 35 Ethylenoxid-Einheiten ethoxyliertes Rizinusöl mit dem INCI-Namen PEG-35 Castor Oil (Cremophor® El) oder mit 40 Ethylenoxid-Einheiten ethoxyliertes hydriertes Rizinusöl mit dem INCI-Namen PEG-40 Hydrogenated Castor Oil (Cremophor® RH 410). Weitere geeignete nichtionische Tenside sind ethoxylierte Fettsäurezuckerester, insbesondere ethoxylierte Sorbitanfettsäureester, die als Polysorbate bekannt sind, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden.

Geeignete kationische Tenside sind weisen eine quaternäre Ammoniumgruppe auf und können durch die allgemeine Formel (I) dargestellt werden,

N⁽⁺⁾R¹R²R³R⁴ X⁽⁻⁾ (I)

wobei R1 bis R4 unabhängig voneinander Alkylgruppen, Arylgruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt, z.B. ein Halogen, Acetat, Phosphat, Nitrat oder Alkylsulfat, vorzugsweise ein Chlorid oder Bromid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide. Besonders bevorzugt ist Cetyltrimethylammoniumchlorid.

In einer weiteren, bevorzugten Ausführungsform enthält das erfindungsgemäße Produkt zusätzlich mindestens ein filmbildendes nichtionisches Polymer. Das nichtionische Polymer ist vorzugsweise in einer Menge von 0,01 bis 15 Gew.%, besonders bevorzugt von 0,5 bis 10 Gew.% enthalten. Es kann sich um ein synthetisches, natürliches oder um ein modifiziertes natürliches Polymer handeln. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Unter filmbildenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar einen Polymerfilm abzuscheiden.

Geeignete synthetische, nichtionische filmbildende Polymere sind Homo- oder Copolymere, die aus mindestens einem der folgenden Monomere aufgebaut sind: Vinylpyrrolidon, Vinylcaprolactam, Vinylester (z.B. Vinylacetat), Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids, Copolymere aus Vinylpyrrolidon und Vinylacetat, Terpolymere aus Vinylpyrrolidon, Vinylacetat und Vinylpropionat, Polyacrylamide, Polyvinylalkohole sowie Polyethylenglykol/Polypropylenglykol Copolymere. Besonders bevorzugt sind Polyvinylpyrrolidon und Copolymere von Vinylpyrrolidon und nichtionischen Comonomeren, z.B. Polyvinylpyrrolidon/Vinylacetat Copolymere. Geeignete natürliche filmbildende Polymere sind z.B. hydroxyalkylierte Polysaccharide, insbesondere Hydroxyalkylcellulose oder Hydroxyalkylguar mit vorzugsweise 2 oder 3 C-Atomen in der Alkylgruppe.

Bei einer besonderen Ausführungsform handelt es sich um ein haarfarbveränderndes Produkt, insbesondere einen Farbfestiger. Die Zusammensetzung enthält dann mindestens einen haarfärbenden Stoff. Hierbei kann es sich um lösliche, organische Farbstoffe, insbesondere um sogenannte direktziehende Farbstoffe oder auch um unlösliche anorganische oder organische Pigmente handeln.

Die Gesamtmenge an Farbstoffen beträgt in dem erfindungsgemäßen Mittel etwa 0,01 bis 7 Gew.%, vorzugsweise etwa 0,2 bis 4 Gew.%. Geeignete direktziehende Farbstoffe sind z.B. Triphenylmethanfarbstoffe, aromatische Nitrofarbstoffe, Azofarbstoffe, Chinonfarbstoffe, kationische oder anionische Farbstoffe. Geeignet sind:
Nitrofarbstoffe (blau) :
   1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Arnino-3-methyl-4-[(2-hydroxyethyl)amino]-6-nitrobenzol (HC Violet No. 1), 4-[Ethyl-(2-hydroxyethyl)-amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-(2-Aminoethylamino)-4-[di(2-hydroxyethyl)amino]-2-nitrobenzol, 4-(Di(2-hydroxyethyl)amino)-2-nitro-1-phenylamino-benzol.
Nitrofarbstoffe (rot):
   1-Amino-4-[(2-hydroxyethyl)amino]-2-nitrobenzol (HC Red No. 7), 2-Amino-4,6-dinitro-phenol, 1,4-Diamino-2-nitrobenzol (CI76070), 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methylamino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)-amino)-benzol, 4-Amino-3-nitrophenol, 4-[(2-Hydroxyethyl)-amino)-3-nitrophenol, 4-[(2-Nitrophenyl)amino]phenol (HC Orange No. 1), 1-[(2-Aminoethyl)amino]-4-(2-hydroxyethöxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Arnino-6-chlor-4-nitrophenol, 4-[(3-Hydroxypropyl)amino]-3-nitrophenol, 2,5-Diamino-6-nitropyridin, 6-Amino-3-((2-hydroxyethyl)amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14).
Nitrofarbstoffe (gelb):
   1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)-amino]-3-nitro-1-trifluormethyl-benzol (HC Yellow No. 6), 1-Chlor-2,4-bis[(2-hydroxyethyl)amino]-5-nitrobenzol (HC Yellow No. 10), 1-Amino-4-((2-aminoethyl)amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitro-benzamid (HC Yellow No. 15) 3-((2-Hydroxyethyl)amino)-4-methyl-1-nitrobenzol, 4-Chlor-3-((2-hydroxyethyl)amino)-1-nitrobenzol.
Chinonfarbstoffe:
   1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-4-methylamino-9,10-anthrachinon (HC Blue No. 8), 1-[(3-Aminopropyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)amino)phenyl)amino)-5-((2-hydroxyethyl)amino)-2,5-cyclohexadien-1,4-dion (HC Green No. 1), 5-Hydroxy-1,4-naphthochinon (CI75500, Natural Brown No. 7), 2-Hydroxy-1,4-naphthochinon (CI75480, Natural Orange No. 6), 1,2-Dihydro-2-(1,3-dihydro-3-oxo-2H-indol-2-yliden)-3H-indol-3-on (CI73000), 4-((5-((2-Hydroxyethyl)amino-1-methyl-1H-pyrazol-4-yl)imino)-4,5-dihydro-5-((2-hydroxyethyl)-imino)-1-methyl-1H-Pyrazol-sulfat(1:1),hydrat(1:1).
Basische Farbstoffe:
   9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid CI42595; Basic Blue No. 7), Di-(4-(dimethylamino)phenyl)-(4-(methyl-phenylamino)-naphthalin-1-yl)carbenium-chlorid (CI42563; Basic Blue No. 8), 3,7-Di(dimethylamino)phenothiazin-5-ium-chlorid (CI52015 Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl]-[4-(methylamino)phenyl]carbenium-chlorid (CI42535; Basic Violet No. 1), Tri(4-amino-3-methylphenyl)carbenium-chlorid (CI42520; Basic Violet No. 2), Tris[4-(dimethylamino)-phenyl]carbenium-chlorid (CI42555; Basic Violet No. 3), 2-[3,6-(Diethylamino)dibenzopyranium-9-yl]-benzoesäurechlorid (CI45170; Basic Violet No. 10), Di(4-aminophenyl)(4-amino-3-methylphenyl)carbeniumchlorid (CI42510 Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 3-[(4-Amino-2,5-dimethoxyphenyl)azo]-N,N,N-trimethylbenzolaminiumchlorid (CI112605, Basic Orange No. 69), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Di[4-(dimethylamino)phenyl]iminomethan-hydrochlorid (CI41000;
   Basic Yellow No. 2), Bis[4-(diethylamino)phenyl]phenyl-carbenium-hydrogensulfat (1:1) (CI42040; Basic Green No. 1), Di(4-(dimethylamino)phenyl)-phenylmethanol (CI42000; Basic Green No. 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid.
Neutrale Azofarbstoffe:
   1-[Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol (Disperse Black No. 9), 4-[(4-Aminophenyl)azo)-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3).
Saure Farbstoffe:
   6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono-und Disulfonsäure) (CI47005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (C145350; Acid Yellow No. 73; D&C Yellow No. 8), 4-((4-Amino-3-sulfophenyl)azo)benzolsulfonsäure-dinatriumsalz (CI13015, Acid Yellow No. 9), 5-[(2,4-Dinitrophenyl)amino]-2-phenylaminobenzolsulfonsäure-natriumsalz (CI10385; Acid Orange No. 3), 4-[(2,4-Dihydroxyphenyl)azo]-benzolsulfonsäure Mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 4-Hydroxy-3-[(2-methoxyphenyl)azo]-1-naphthalinsulfonsäure-mononatriumsalz (CI14710; Acid Red No. 4), 6-Hydroxy-5-[(4-sulfonaphth-1-yl)azo]-2,4-naphthalin-disulfonsäuretrinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430; Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (CI45380 Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (CI15685; Acid Red No. 184), (2-Sulfophenyl)di[4-(ethyl((4-sulfophenyl)methyl)amino)phenyl]-carbenium-dinatriumsalz Betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxy-naphth-1-yl)carbenium-inneres Salz Mononatriumsalz (CI44090; Food Green No. 4; Acid Green No. 50), Bis[4-(diethylamino)-phenyl](2,4-disulfophenyl)carbenium-inneres salz, Natriumsalz (2:1) (CI42045; Food Blue No. 3; Acid Blue No. 1), Bis [4- (diethylamino)phenyl] (5-hydroxy-2,4-disulfophenyl)-carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 1-Amino-4-(phenylamino)-9,10-anthrachinon-2-sulfonsäure (CI62055; Acid Blue No. 25), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-I(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)-azo]-3-(phenylazo)-2,7-naphthalin-disulfonsäure Dinatriumsalz (CI20470; Acid Black No. 1), 3-Hydroxy-4-[(2-hydroxynaphth-1-yl)azo]-7-nitro-1-naphthalin-sulfonsäure-chrom-komplex (3:2) (CI15711; Acid Black No. 52), 3-[(2,4-Dimethyl-5-sulfophenyl)azo)-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195).

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Geeignete haarfärbende Pigmente sind im Anwendungsmedium praktisch unlösliche Farbmittel und können anorganisch oder organisch sein. Auch anorganisch-organische Mischpigmente sind möglich. Bei den Pigmenten handelt es sich vorzugsweise nicht um Nanopigmente. Die bevorzugte Teilchengröße beträgt 1 bis 200 µm, insbesondere 3 bis 150 µm, besonders bevorzugt 10 bis 100 µm.Bevorzugt sind anorganische Pigmente. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal). Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt ist. Derartige Pigmente werden beispielsweise unter den Handelsbezeichnung Rona®, Colorona®, Dichrona® und Timiron® von der Firma Merck, Deutschland vertrieben. Organische Pigmente sind z.B. die natürlichen Pigmente Sepia, Gummigutt, Knochenkohle, Kasseler Braun, Indigo, Chlorophyll und andere Pflanzenpigmente. Synthetische organische Pigmente sind z.B. AzoPigmente, Anthrachinoide, Indigoide, Dioxazin-, Chinacridon- , Phtalocyanin-, Isoindolinon-, Perylen- und Perinon-, Metallkomplex-, Alkaliblau- und Diketopyrrolopyrrolpigmente.

Das erfindungsgemäße Produkt kann darüber hinaus die für Haarbehandlungsmittel üblichen zusatzbestandteile enthalten, z.B. Parfümöle in einer Menge von 0,01 bis 0,5 Gew.%; Konservierungsmittel wie z.B. Parabene in einer Menge von 0,01 bis 1,0 Gew.%; Puffersubstanzen, wie z.B. Natriumcitrat oder Natriumphosphat, in einer Menge von 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, direktziehende Haarfarbstoffe, Glanzgeber und Kämmbarkeitsverbesserer in einer Menge von je etwa 0,01 bis 2 Gew.%.

Das erfindungsgemäße Aerosolschaumprodukt wird in einer druckfesten Aerosolverpackung abgefüllt, welche mit einer Vorrichtung zum Verschäumen (Aersolschaumkopf) versehen wird. Beim Ausbringen aus diesr Aerosolverpackung bildet sich ein Schaum, welcher leicht in das Haar eingearbeitet werden kann und beim Einarbeiten auf dem Haar schnell zusammenbricht.

Das erfindungsgemäße kosmetische Mittel wird angewendet, indem es in einer zur Erzielung des gewünschten haarpflegenden oder haarfestigenden Effektes ausreichenden Menge auf feuchtes, handtuchtrockenes Haar aufgetragen und eingearbeitet wird und ohne Ausspülen im Haar belassen wird. Anschließend kann die Frisur in üblicher Weise geformt werden bzw. können die Haare eingelegt und zum Schluß trocken gefönt werden. Es ist aber auch möglich, das Mittel direkt auf trockenem Haar anzuwenden.

Es wird ein Schaum mit fadenziehenden Eigenschaften und guter, kompakter Schaumqualität erhalten, der auf dem Haar schnell zusammenbricht und gut in das Haar einarbeitbar ist. Er zeichnet sich aus durch gute Föneigenschaften, d.h. durch ein besonders gutes Gleitvermögen einer Haarbürste während des Trockenfönens von feuchtem, behandeltem Haar, einen angenehmen, glatten Griff des feuchten und des trockenen Haares, gute Elastizität, keine Belastung, einen schönen Glanz und starke Festigung.

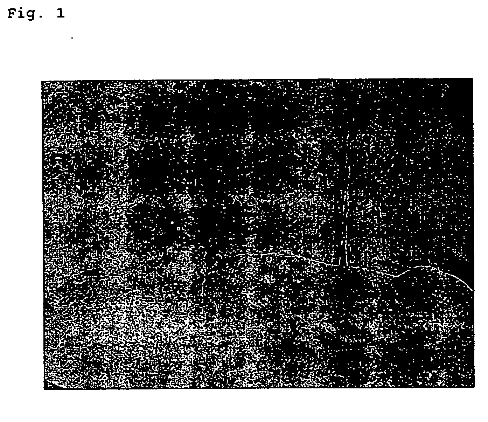

Figur 1 zeigt eine fotographische Aufnahme der fädenziehenden Eigenschaft einer verschäumten erfindungsgemäßen Zusammensetzung gemäß Beispiel 1A.

Die nachfolgenden Beispiele sollen den Gegenstand der Erfindung näher erläutern.

### Beispiele

Die nachfolgend beschriebenen Wirkstoffmischungen wurden jeweils im Verhältnis 92:8 mit Propan/Butan 4.8 bar als Treibmittel in einer Aerosoldose mit Schaumventil abgefüllt. Die beschriebenen Schaumeigenschaften beziehen sich auf frisch hergestellte Schäume, unmittelbar nach Abgabe aus der Aerosolverpackung.

Die Beurteilungen der Schaumqualitäten und Anwendungseigenschaften erfolgte in Salonversuchen, wobei Testpersonen mit jeweils gleichen Schaummengen behandelt wurden. Die Schäume sowie die mit den Schäumen behandelten Haare wurden durch ausgebildete Friseurfachkräfte sensorisch beurteilt.

### Beispiel 1: Wirkstoffmischung für Aerosol-Haarschaum

| | A [g] | B [g] | C [g] |
|---|---|---|---|
| (A) Merquat® 550 ¹⁾ | 6,9 | 13,6 | 2,27 |
| (B) Luviflex® Soft ²⁾ | 5,6 | 3,73 | 6,5 |
| Aminomethylpropanol (95%ig) | 0,9 | 0,6 | 1,0 |
| Laureth-4 | 0,2 | 0,2 | 0,2 |
| Cetyltrimethylammoniumchlorid | 0,15 | 0,15 | 0,15 |
| Ethanol | 10 | 10 | 10 |
| Wasser | Ad 100 | Ad 100 | Ad 100 |
| Feststoffmengenverhältnis (A) : (B) | 1:3 | 1:1 | 1:11 |

| | | | |
|---|---|---|---|
| ¹⁾ Polyquaternium-7, Dimethyldiallylammoniumchlorid/Acrylamid Copolymer, 8%ig in Wasser | | | |
| ²⁾ Acrylates Copolymer, Ethylacrylat/Methacrylsäure Copolymer, 30%ig in Wasser | | | |

Schaumqualität Schaum 1A: sehr kompakt, fädenziehend Schaumqualität Schaum 1B: weniger fädenziehend, schleimiger, schmieriger

### Anwendungseigenschaften Schaum 1A:

glatter Griff im feuchten Haar, gute Feuchtkämmbarkeit, gute Föneigenschaften bei Fönfrisuren, gute Formerstellung bei Lockenfrisuren, schön definiertes Lockenbild bei Lockenfrisuren, einsetzende Klebephase beim Trocknen, schöner glatter Griff, nicht belastend, gute Elastizität, sehr schöner Glanz.

Schaum IC: weniger fädenziehend, Kämmbarkeit schlechter, weniger Elastizität im trockenen Haar, weniger Definition der Haare, schwächere Festigung, ansonsten gleichwertig zu Schaum 1A.

### Beispiel 2: Vergleichsrezepturen

| | A [g] | B [g] | C [g] | D [g] |
|---|---|---|---|---|
| (A') Celquat® L200 ³⁾ | 0,55 | 1,1 | - | - |
| (A') Luviquat® HM 552 ⁴⁾ | - | - | 2,75 | 5,6 |
| (B) Luviflex® Soft | 5,6 | 3,73 | 5,6 | 3,73 |
| Aminomethylpropanol (95%ig) | 0,9 | 0,6 | 0,9 | 0,6 |
| Ethanol | 10 | 10 | 10 | 10 |
| Laureth-4 | 0,2 | 0,2 | 0,2 | 0,2 |
| Cetyltrimethylammoniumchlorid | 0,15 | 0,15 | 0,15 | 0,15 |
| Wasser | Ad 100 | Ad 100 | Ad 100 | Ad 100 |
| Feststoffmengenverhältnis (A'):(B) | 1:3 | 1:1 | 1:3 | 1:1 |

| | | | | |
|---|---|---|---|---|
| ³⁾ Polyquaternium-4, Dimethyldiallylammoniumchlorid/Hydroxyethylcellulose Copolymer | | | | |
| ⁴⁾ Polyquaternium-16, Vinylimidazoliummethochlorid/Vinylpyrrolidon Copolymer, 20%ig in Wasser | | | | |

### Beurteilung der Schäum 2A und 2B im Vergleich zu Schaum 1A:

Schlechte Föneigenschaften, erschwerte Formerstellung der Haare, stumpfes und glanzloses Aussehen der Haare, höhere Belastung der Haare

### Beurteilung der Schäum 2C und 2D im Vergleich zu Schaum 1A:

Sehr schlechte Föneigenschaften, Aussehen der Haare im Trockenen matt und stumpf, klebrige und unangenehme Belastung der Haare.

### Beispiel 3: Wirkstoffmischung für Aerosol-Schaumfestiger

| | |
|---|---|
| 6,9 g | Merquat® 550 |
| 5,6 g | Luviflex® Soft |
| 0,9 g | Aminomethylpropanol (95%ig) |
| 1,5 g | Vinylpyrrolidon/Vinylacetat Copolymer (Luviskol® VA64) |
| 0,2 g | Laureth-4 |
| 0,3 g | Parfüm |
| 0,15 g | Cetyltrimethylammoniumchlorid |
| 10 g | Ethanol |
| ad 100 g | Wasser |

Die Wirkstoffmischung wird im Verhältnis 92:8 mit Propan/Butan 4.8 bar als Treibmittel in einer Aerosoldose mit Schaumventil abgefüllt.

### Beispiel 4: Farb-Schaumfestiger

| | |
|---|---|
| 6,9 g | Merquat® 550 |
| 5,6 g | Luviflex® Soft |
| 0,9 g | Aminomethylpropanol (95%ig) |
| 0,2 g | Laureth-4 |
| 0,2 g | Parfüm |
| 0,11 g | 3-(((2-Nitro-4-(trifluormethyl)phenyl)amino)-1,2-propandiol |
| 0,15 g | Cetyltrimethylammoniumchlorid |
| 10 g | Ethanol |
| ad 100 g | Wasser |

Die Wirkstoffmischung wird im Verhältnis 94:6 mit Propan/Butan 5 bar als Treibmittel in einer Aerosoldose mit Schaumventil abgefüllt.

### Beispiel 5: Farbmousse rot

| | |
|---|---|
| 5,00 g | Timiron® starlight red (Merck) ¹⁾ |
| 6,9 g | Merquat® 550 |
| 5,6 g | Luviflex® Soft |
| 0,9 g | Aminomethylpropanol (95%ig) |
| 0,2 g | Laureth-4 |
| 0,2 g | Parfüm |
| 0,15 g | Cetyltrimethylammoniumchlorid |
| 10 g | Ethanol |
| ad 100 g | Wasser |

| | |
|---|---|
| ¹⁾ Mica/Titandioxid-Pigment mit roter Reflektionsfarbe | |

Die Wirkstoffmischung wird im Verhältnis 94:6 mit Propan/Butan 5 bar als Treibmittel in einer Aerosoldose mit Schaumventil abgefüllt.

## Patentansprüche

1. Aerosolschaumprodukt zur Haarbehandlung, umfassend eine verschäumbare Zusammensetzung mit einem Gehalt an
(A) mindestens einem Dialkyldiallylammoniumchlorid/Acrylamid Copolymer,
(B) mindestens einem Copolymer, gebildet aus einer ersten Monomerart, die ausgewählt ist aus Alkylacrylaten und Alkylmethacrylaten und mindestens einer zweiten Monomerart, die ausgewählt ist aus Acrylsäure und Methacrylsäure,
(C) mindestens einer Base zur teilweisen oder vollständigen Neutralisation des Copolymers (B) und
(D) einem geeigneten Lösungsmittelsystem,
wobei die Zusammensetzung zusammen mit mindestens einem Treibmittel (E) in einer druckfesten Verpackung abgefüllt ist.

2. Schaumprodukt nach Anspruch 1, **dadurch gekennzeichnet, dass** das Copolymer (A) ein Dimethyldiallylammoniumchlorid/Acrylamid Copolymer ist.

3. Schaumprodukt nach Anspruch 2, **dadurch gekennzeichnet, dass** das Copolymer (A) eine kationische Ladungsdichte, ausgedrückt als Molekulargewicht pro kationische Ladung im Bereich von 200 bis 250 aufweist.

4. Schaumprodukt nach einem der vorhergehenden Ansprüche, **dadurch gekennzeichnet, dass** das Copolymer (B) ein Alkylacrylat/Methacrylsäure Copolymer ist, wobei die Alkylgruppe 1 bis 4 C-Atome aufweist.

5. Schaumprodukt nach einem der vorhergehenden Ansprüche, **dadurch gekennzeichnet, dass** das Verhältnis der Anzahl negativer Ladungen des Copolymers (B) zur Anzahl positiver Ladungen des Copolymer (A) im Bereich von 1:1 bis 10:1 liegt.

6. Schaumprodukt nach einem der vorhergehenden Ansprüche, **dadurch gekennzeichnet, dass** die Base (C) ausgewählt ist aus Aminoalkylalkoholen.

7. Schaumprodukt nach einem der vorstehenden Ansprüche, **dadurch gekennzeichnet, dass** zusätzlich ein Tensid (F) enthalten ist.

8. Schaumprodukt nach Anspruch 7, **dadurch gekennzeichnet, dass** das Tensid (F) ausgewählt ist aus
- nichtionischen Tensiden, ausgewählt aus Fettsäureglyceridethoxylaten, Fettalkoholethoxylaten, Fettaminethoxylaten, Fettsäurealkanolamidethoxylaten, Fettsäureesterethoxylaten, ethoxylierten Fettsäurezuckerestern, ethoxylierten Sorbitanfettsäureestern, nicht-ethoxylierten Fettsäurezuckerestern und Alkylpolyglycosiden und
- kationischen Tensiden der allgemeinen Formel (I)
N⁽⁺⁾R¹R²R³R⁴ X⁽⁻⁾ (I)
wobei R1 bis R4 unabhängig voneinander Alkylgruppen, Arylgruppen, Alkoxygruppen, Polyoxyalkylengruppen, Alkylamidogruppen, Hydroxyalkylgruppen oder Alkarylgruppen mit 1 bis 22 C-Atomen bedeuten und X⁽⁻⁾ ein Anion darstellt.

9. Schaumprodukt nach einem der vorstehenden Ansprüche, **dadurch gekennzeichnet, dass** das Treibmittel (E) ausgewählt ist aus Propan, i-Butan, n-Butan oder Dimethylether oder aus Mischungen dieser Treibgase.

10. Schaumprodukt nach einem der vorstehenden Ansprüche, **dadurch gekennzeichnet, dass** das Copolymer (A) in einer Menge von 0,01 bis 10 Gew.%, das Copolymer (B) in einer Menge von 0,1 bis 10 Gew.%, die Base (C) in einer Menge, die ausreichend ist, um das Copolymer (B) zu 50 bis 100% zu neutralisieren, das Treibmittel (E) in einer Menge von 1 bis 20 Gew.%, das Lösungsmittelsystem (D) in einer Menge von 50 bis 98 Gew.% und das Tensid (F) in einer Menge von 0 bis 15 Gew.% enthalten ist.

11. Schaumprodukt nach einem der vorstehenden Ansprüche, **dadurch gekennzeichnet, dass** es zusätzlich mindestens ein nichtionisches filmbildendes Polymer enthält.

12. Schaumprodukt nach Anspruch 11, **dadurch gekennzeichnet, dass** das nichtionische Polymer ausgewählt ist aus Polyvinylpyrrolidon und Copolymeren von Vinylpyrrolidon und nichtionischen Comonomeren.

13. Schaumprodukt nach einem der vorstehenden Ansprüche, **dadurch gekennzeichnet, dass** es zusätzlich mindestens einen haarfärbenden Stoff enthält.

14. Schaumprodukt nach einem der vorstehenden Ansprüche, **dadurch gekennzeichnet, dass** es einen Gehalt aufweist an
(A) 0,1 bis 5 Gew.% Dimethyldiallylammoniumchlorid/Acrylamid Copolymer,
(B) 0,1 bis 5 Gew.% Ethylacrylat/Methacrylsäure Copolymer,
(C) einer Base in einer Menge, die ausreichend ist, um das Copolymer (B) zu 50 bis 100% zu neutralisieren,
(D) 50-98 Gew.% eines Lösungsmittels, ausgewählt aus Wasser, C2-3-Alkoholen oder deren Gemisch,
(E) 1 bis 20 Gew.% eines Treibmittels ausgewählt aus C3- und C4-Kohlenwasserstoffen,
wobei das Gewichtsverhältnis des Copolmyers (B) zum Copolymer (A) von größer 1:1 bis kleiner 10:1 ist.

15. Verwendung einer Zusammensetzung mit einem Gehalt an
(A) mindestens einem Dialkyldiallylammoniumchlorid/Acrylamid Copolymer,
(B) mindestens einem Copolymer, gebildet aus einer ersten Monomerart, die ausgewählt ist aus Alkylacrylaten und Alkylmethacrylaten und mindestens einer zweiten Monomerart, die ausgewählt ist aus Acrylsäure und Methacrylsäure,
(C) mindestens einer Base zur teilweisen oder vollständigen Neutralisation des Copolymers (B) und
(D) einem geeigneten Lösungsmittelsystem zur Behandlung oder Herstellung gelockter oder gewellter Haare.

## Claims

1. Aerosol foam product for hair treatment, comprising a foamable composition containing
(A) at least one dialkyldiallylammonium chloride/acrylamide copolymer,
(B) at least one copolymer formed from a first kind of monomer selected from alkyl acrylates and alkyl methacrylates and at least one second kind of monomer selected from acrylic acid and methacrylic acid,
(C) at least one base for partial or total neutralization of the copolymer (B), and
(D) a suitable solvent system,
the composition being filled together with at least one propellant (E) in a pressure-resistant container.

2. Foam product according to Claim 1, **characterized in that** the copolymer (A) is a dimethyldiallylammonium chloride/acrylamide copolymer.

3. Foam product according to Claim 2, **characterized in that** the copolymer (A) has a cationic charge density in the range from 200 to 250, expressed as molecular weight per unit cationic charge.

4. Foam product according to any one of the preceding claims, **characterized in that** the copolymer (B) is an alkyl acrylate/methacrylic acid copolymer, the alkyl group containing 1 to 4 carbon atoms.

5. Foam product according to any one of the preceding claims, **characterized in that** the ratio of the number of negative charges of the copolymer (B) to the number of positive charges of the copolymer (A) is in a range of 1:1 to 10:1.

6. Foam product according to any one of the preceding claims, **characterized in that** the base (C) is selected from aminoalkyl alcohols.

7. Foam product according to any one of the above claims, **characterized in that** additionally a surfactant (F) is included.

8. Foam product according to Claim 7, **characterized in that** the surfactant (F) is selected from
- non-ionic surfactants, selected from fatty acid glyceride ethoxylates, fatty alcohol ethoxylates, fatty amine ethoxylates, fatty acid alkanol amide ethoxylates, fatty acid ester ethoxylates, ethoxylated fatty acid sugar esters, ethoxylated sorbitan fatty acid esters, non-ethoxylated fatty acid sugar esters and alkylpolyglycosides, and
- cationic surfactants of the general formula (I)
N⁽⁺⁾R¹R²R³R⁴ X⁽⁻⁾ (I)
where R¹ to R⁴ independently of each other represent alkyl groups, aryl groups, alkoxy groups, polyoxyalkylene groups, alkylamido groups, hydroxyalkyl groups or alkaryl groups with 1 to 22 carbon atoms, and X⁽⁻⁾ represents an anion.

9. Foam product according to any one of the above claims, **characterized in that** the propellant (E) is selected from propane, i-butane, n-butane or dimethyl ether or from mixtures of these propellant gases.

10. Foam product according to any one of the above claims, **characterized in that** the copolymer (A) is present in an amount from 0.01% to 10% by weight, copolymer (B) in an amount from 0.1% to 10% by weight, base (C) in an amount sufficient to neutralize from 50% to 100% of copolymer (B), propellant (E) in an amount from 1% to 20% by weight, solvent system (D) in an amount from 50% to 98% by weight, and surfactant (F) in an amount from 0 to 15% by weight.

11. Foam product according to any one of the above claims, **characterized in that** it further comprises at least one non-ionic film-forming polymer.

12. Foam product according to Claim 11, **characterized in that** the non-ionic polymer is selected from polyvinylpyrrolidone and copolymers of vinylpyrrolidone and nonionic comonomers.

13. Foam product according to any one of the above claims, **characterized in that** it further comprises at least one hair-dyeing substance.

14. Foam product according to any one of the above claims, **characterized in that** it contains
(A) 0.1% to 5% by weight of dimethyldiallylammonium chloride/acrylamide copolymer,
(B) 0.1% to 5% by weight of ethyl acrylate/methacrylic acid copolymer,
(C) a base in an amount sufficient for neutralizing from 50% to 100% of copolymer (B),
(D) 50-98% by weight of a solvent selected from water, C2-3 alcohols or a mixture thereof,
(E) 1% to 20% by weight of a propellant selected from C3 and C4 hydrocarbons,
the weight ratio of copolymer (B) to copolymer (A) being from greater than 1:1 to less than 10:1.

15. Use of a composition containing
(A) at least one dialkyldiallylammonium chloride/acrylamide copolymer,
(B) at least one copolymer formed from a first kind of monomer selected from alkyl acrylates and alkyl methacrylates and at least one second kind of monomer selected from acrylic acid and methacrylic acid,
(C) at least one base for partial or total neutralization of the copolymer (B), and
(D) a suitable solvent system
to treat or make curled or waved hair.

## Revendications

1. Produit mousseux en aérosol pour le traitement des cheveux, comprenant une composition moussante présentant une teneur en
(A) au moins un copolymère de chlorure de dialkyldiallylammonium/acrylamide,
(B) au moins un copolymère formé à partir d'un premier type de monomères, choisi parmi les acrylates d'alkyle et les méthacrylates d'alkyle et d'au moins un deuxième type de monomères, choisi parmi l'acide acrylique et l'acide méthacrylique,
(C) au moins une base pour la neutralisation partielle ou complète du copolymère (B) et
(D) un système de solvant approprié
la composition étant introduite avec au moins un agent propulseur (E) dans un emballage résistant à la pression.

2. Produit mousseux selon la revendication 1, **caractérisé en ce que** le copolymère (A) est un copolymère de chlorure de diméthyldiallylammonium/acrylamide.

3. Produit mousseux selon la revendication 2, **caractérisé en ce que** le copolymère (A) présente une densité de charge cationique, exprimée comme poids moléculaire par charge cationique dans la plage de 200 à 250.

4. Produit mousseux selon l'une quelconque des revendications précédentes, **caractérisé en ce que** le copolymère (B) est un copolymère d'acrylate d'alkyle/acide méthacrylique, le groupe alkyle présentant 1 à 4 atomes de carbone.

5. Produit mousseux selon l'une quelconque des revendications précédentes, **caractérisé en ce que** le rapport du nombre de charges négatives du copolymère (B) au nombre de charges positives du copolymère (A) se situe dans la plage de 1:1 à 10:1.

6. Produit mousseux selon l'une quelconque des revendications précédentes, **caractérisé en ce que** la base (C) est choisie parmi les alcools aminoalkyliques.

7. Produit mousseux selon l'une quelconque des revendications précédentes, **caractérisé en ce qu'**un agent tensioactif (F) est en outre contenu.

8. Produit mousseux selon la revendication 7, **caractérisé en ce que** l'agent tensioactif (F) est choisi parmi
- les agents tensioactifs non ioniques, choisis parmi les éthoxylates de glycérides d'acide gras, les éthoxylates d'alcool gras, les éthoxylates d'amine grasse, les éthoxylates d'alcanolamides d'acide gras, les éthoxylates d'esters d'acide gras, les esters éthoxylés de sucres d'acide gras, les esters éthoxylés d'acide gras de sorbitane, les esters non éthoxylés de sucres d'acide gras et les alkylpolyglycosides et
- les agents tensioactifs cationiques de formule générale (I)
N⁽⁺⁾R¹R²R³R⁴ X⁽⁻⁾ (I)
où R¹ à R⁴ signifient, indépendamment l'un de l'autre, des groupes alkyle, aryle, alcoxy, polyoxyalkylène, alkylamido, hydroxyalkyle ou alkaryle, comprenant 1 à 22 atomes de carbone et X⁽⁻⁾ représente un anion.

9. Produit mousseux selon l'une quelconque des revendications précédentes, **caractérisé en ce que** l'agent propulseur (E) est choisi parmi le propane, l'i-butane, le n-butane ou le diméthyléther ou parmi les mélanges de ces gaz propulseurs.

10. Produit mousseux selon l'une quelconque des revendications précédentes, **caractérisé en ce que** le copolymère (A) est contenu en une quantité de 0,01 à 10% en poids, le copolymère (B) est contenu en une quantité de 0,1 à 10% en poids, la base (C) est contenue en une quantité qui est suffisante pour neutraliser le copolymère (B) à raison de 50 à 100%, l'agent propulseur (E) est contenu en une quantité de 1 à 20% en poids, le système de solvant (D) est contenu en une quantité de 50 à 98% en poids et l'agent tensioactif (F) est contenu en une quantité de 0 à 15% en poids.

11. Produit mousseux selon l'une quelconque des revendications précédentes, **caractérisé en ce qu'**il contient en outre au moins un polymère non ionique, formant un film.

12. Produit mousseux selon la revendication 11, **caractérisé en ce que** le polymère non ionique est choisi parmi la polyvinylpyrrolidone et les copolymères de vinylpyrrolidone et de comonomères non ioniques.

13. Produit mousseux selon l'une quelconque des revendications précédentes, **caractérisé en ce qu'**il contient en outre au moins une substance colorant les cheveux.

14. Produit mousseux selon l'une quelconque des revendications précédentes, **caractérisé en ce qu'**il contient une teneur
(A) de 0,1 à 5% en poids en copolymère de chlorure de diméthyldiallylammonium/acrylamide,
(B) de 0,1 à 5% en poids en copolymère d'acrylate d'éthyle/acide méthacrylique,
(C) en base en une quantité qui est suffisante pour neutraliser le copolymère (B) à raison de 50 à 100%,
(D) de 50-98% en poids en solvant, choisi parmi l'eau, les alcools en C₂ - C₃ ou leur mélange,
(E) de 1 à 20% en poids en agent propulseur, choisi parmi les hydrocarbures en C₃ et C₄,
le rapport pondéral du copolymère (B) au copolymère (A) étant supérieur à 1:1 et inférieur à 10:1.

15. Utilisation d'une composition présentant une teneur en
(A) au moins un chlorure de dialkyldiallylammonium/acrylamide,
(B) au moins un copolymère formé à partir d'un premier type de monomères, choisi parmi les acrylates d'alkyle et les méthacrylates d'alkyle et d'au moins un deuxième type de monomères, choisi parmi l'acide acrylique et l'acide méthacrylique
(C) au moins une base pour la neutralisation partielle ou complète du copolymère (B) et
(D) un système de solvant approprié
pour le traitement ou la réalisation de cheveux bouclés ou ondulés.
